# EUROPEAN PATENT APPLICATION

(11) **EP 3 000 882 A1**
(43) Date of publication of application: **30.03.2016**
(21) Application number: 14801154.7
(22) Date of filing: 15.05.2014
(51) Int. Cl.: C12N 15/09, C12Q 1/68, G01N 33/53

(54) **NUCLEIC ACID ANALYSIS KIT AND NUCLEIC ACID ANALYSIS METHOD**

(30) Priority: 22.05.2013 JP 2013108142
(71) Applicant: Olympus Corporation, Shibuya-ku Tokyo 151-0072 (JP)
(72) Inventor: HORI, Kunio, Tokyo 151-0072 (JP)
(74) Representative: Schicker, Silvia
(86) International application number: PCT/JP2014/062917
(87) International publication number: WO 2014/188941

(57) **Abstract**

A number of samples provided for analysis are identified correctly by making use of smaller kinds of nucleic acids. Provided is a nucleic acid analysis kit (100) having a plurality of supports (2) that respectively retain identifier probes (1) containing a nucleic acid and support specimens. The identifier probes (1) contains at least one kind of a nucleic acid having a known base sequence, differ in at least one of the kind and amount of the nucleic acid for each of the supports (2), and are retained by the supports (2) so as to be mixed with the specimens, respectively.

## Description

### [Technical Field]

The present invention relates to a nucleic acid analysis kit and a nucleic acid analysis method.

### [Background Art]

In conventional nucleic acid analysis, during a period between collection of specimens and provision of a nucleic acid contained in the specimens to analysis, a vessel has been replaced frequently in the pretreatment for extracting a nucleic acid from the specimens. When the pretreatment is performed manually, it is the common practice to prevent misidentification of samples by associating a vessel before replacement with a vessel after replacement whenever vessels are replaced. Due to the cumbersome association work of vessels, the larger the number of specimens, the higher the occurrence probability of misidentification of samples.

As means for preventing this misidentification of samples, there is known a method of mixing an identifier probe in a specimen and amplifying the identifier probe together with the specimen (refer to, for example, Patent Document 1). This identifier probe has, in a region to be amplified by the same primer as that for the specimen, a base sequence decodable to an individual code given to the specimens. It is therefore possible, after amplification of specimens, to identify which specimen the amplified product has been derived from by decoding the individual code of an identifier probe contained in the amplified product.

On the other hand, as a method of analyzing the amount or space distribution of activity of a specific molecule contained in a section of a biological tissue, there is known a method of introducing probes capable of recognizing a target molecule at a plurality of positions on the section and adding to the probes a tag for identifying the position of the probe on the section (refer to, for example, Patent Document 2). By collecting from the section the probe that has reacted with the molecule and analyzing the tag added to the probe, it is possible to know the amount or activity of the target molecule at each position of the section.

### [Citation List]

### [Patent Literature]

[PLT 1] Japanese Patent Laid-Open No. 2007-74967
[PLT 2] US Patent Application Publication No. 2011/0245111

### [Summary of Invention]

### [Technical Problem]

The methods described in Patent Documents 1 and 2 however require preparation of an identifier probe having a basic sequence or a tag for each specimen. This means that when the number of specimens to be analyzed is large, these methods are disadvantageous because many kinds of identifier probes or tags should be prepared.

With the foregoing in view, the present invention has been made. An object of the present invention is to provide a nucleic acid analysis kit and a nucleic analysis method capable of correctly identifying the origin of each of a number of samples provided for analysis by making use of smaller kinds of nucleic acids.

### [Solution to Problem]

With a view to achieving the above-mentioned object, the present invention provides the following means.

In a first embodiment of the present invention, there is provided a nucleic acid analysis kit having a plurality of supports which retain identifier probes containing a nucleic acid and support specimens, respectively. The identifier probes each contain at least one kind of a nucleic acid having a known base sequence, are different from one another in at least one of the kind and amount of the nucleic acid for each of the supports, and are retained by the supports so as to be miscible with the specimens respectively.

According to the first embodiment of the present invention, when the specimens are supported by the plurality of supports, respectively, the nucleic acid for identification contained in the identifier probes supported by the supports is mixed with each of the specimens. At this time, at least one of the kind and amount of the nucleic acid for identification to be mixed with each of the specimens is different for each of the specimens. When a sample prepared from each of the specimens supported by the supports is subjected to nucleic acid analysis, by analyzing also the nucleic acid for identification contained in each of the samples and identifying also the kind and/or amount of the nucleic acid, it is possible to correctly identify the original specimen of each of the samples based on the kind and/or amount of the nucleic acid for identification.

A number of specimens can be distinguished from one another by differentiating at least one of the kind and amount of the nucleic acid for identification to be mixed with each of the specimens so that each of the identifier probes is only required to contain smaller kinds of nucleic acids. The origin of each of a number of samples can be correctly identified by making use of smaller kinds of nucleic acids.

In the above-mentioned first embodiment, the nucleic acid analysis kit may be equipped with a correspondence table in which each of the plurality of supports is associated with the composition of the identifier probes retained respectively by the plurality of supports, that is, combination of the kind and amount of the nucleic acid.

This makes it possible to easily identify the support and the composition of the identifier probe retained by the support.

In the above-mentioned first embodiment, the supports are vessels having therein the specimens, respectively, and the identifier probes may be sealed in the vessels, respectively.

This makes it possible to easily mix each of the identifier probes with each of the specimens only by placing the specimen in the vessel.

In the above-mentioned first embodiment, the kit is equipped with a substrate which can be divided into a plurality of chips along predetermined division lines and to which a section of a biological tissue can be attached as the specimen; and the identifier probe may be retained by each of the plurality of chips.

This makes it possible to, upon analyzing a portion of the section of a biological tissue as the specimen, simultaneously divide the section and a substrate to which the section has been attached along the division line and thereby prepare a fragment of the section having a single kind of the identifier probe mixed therein.

In the above-mentioned first embodiment, the nucleic acid may be a DNA (deoxyribonucleic acid).

This makes it possible to provide the kit with a structure suited for analysis of the DNA contained in each of the specimens.

In the above-mentioned first embodiment, the nucleic acid may be an RNA (ribonucleic acid).

When the nucleic acid of the specimen to be analyzed is an RNA, the nucleic acid contained in each of the identifier probes is desirably an RNA. When the identifier probes each contain an RNA, a modified RNA, for example, an RNA obtained by substituting the 2' position of an oligonucleotide with a methyl group may be used in order to prevent degradation of the RNA with a ribonuclease.

In the above-mentioned first embodiment, the nucleic acid may be a nucleic acid analog. Examples of the nucleic acid analog include substances obtained by modifying the side chain or the like of a natural nucleotide (naturally occurring nucleotide) such as DNA or RNA with a functional group such as amino group and substances obtained by labeling with a protein, a low molecular compound, or the like. More specific examples include bridged nucleic acid (BNA), 2'-O,4'-C-ethylene-bridged nucleic acids (ENA), nucleotide obtained by substituting the 4'-oxygen atom of a naturally occurring nucleotide with a sulfur atom, nucleotide obtained by substituting the 2'-hydroxyl group of a naturally occurring ribonucleotide with a methoxy group, hexitol nucleic acid (HNA), and peptide nucleic acid (PNA).

In the above-mentioned first embodiment, the DNA has a base length of 20 bases or more but not more than 500 bases.

This makes it possible to facilitate handling of the DNA in the preparation of the identifier probes, nucleic acid analysis, or pretreatment.

In the above-mentioned first embodiment, the DNA retained by each of the supports may have 100 molecules or more but not more than 100,000 molecules.

This make it possible to secure the quantitative accuracy of the DNA of each of the identifier probes while preventing the identifier probes from influencing on the analysis of a nucleic acid contained in each of the specimens.

In a second embodiment of the present invention, there is provided a nucleic acid analysis method including a probe adding step in which identifier probes each containing at least one nucleic acid having a known base sequence are added to a plurality of specimens, respectively; and a nucleic acid analyzing step in which the nucleic acid contained in each of the specimens is analyzed. In the probe adding step, the identifier probes different from each other in at least one of the kind and amount of the nucleic acid are added to the plurality of specimens, respectively, while in the nucleic acid analyzing step, the nucleic acid contained in each of the identifier probes added to the specimens is also analyzed.

In the second embodiment of the present invention, when after respectively adding the identifier probes to the specimens, nucleic acid analysis of samples prepared from the specimens is performed in the nucleic acid analyzing step, both analysis of the nucleic acid for identification contained in each of the samples and identification of the kind and/or amount of the nucleic acid are performed. Then, the original specimen of each of the samples can be identified correctly based on the kind and/or amount of the nucleic acid for identification.

By differentiating at least one of the kind and amount of the nucleic acid for identification to be added to each of the specimens, a large number of specimens can be distinguished from one another. It is therefore only necessary that the identifier probes each contain smaller kinds of nucleic acids. The origin of each of a large number of samples can therefore be identified correctly by making use of smaller kinds of nucleic acids.

In the above-mentioned second embodiment, the specimens may each be added to a vessel having each of the identifier probes therein in the probe adding step.

This facilitates addition of each of the identifier probes to each of the specimens only by placing the specimen in the vessel.

In the above-mentioned second embodiment, in the probe adding step, a section of a biological tissue may be attached onto a substrate having the identifier probes attached thereto in scattered form, and may further include a dividing step in which the substrate having the section attached thereto is divided, together with the section, into a plurality of chips each containing one of the identifier probes. In the nucleic acid analyzing step, a portion of the section attached to each of the chips obtained in the dividing step may be analyzed.

This makes it possible to easily analyze the nucleic acids present at each position of the section.

In the above-mentioned second embodiment, in the probe adding step, the identifier probes may be attached in scattered form onto a section of a biological tissue, may further include a dividing step in which the section having the identifier probes attached thereto is divided into a plurality of fragments containing one of the identifier probes and in the nucleic acid analyzing step, the fragments of the section cut in the dividing step may be analyzed. This makes it possible to easily analyze the nucleic acid present at each of the positions of the section.

In the above-mentioned second embodiment, the method further includes, prior to the dividing step, a hybridization step in which the section is subjected to in situ hybridization using a nucleic acid probe having a known base sequence complementary to a target nucleic acid.

This makes it possible to detect the target nucleic acid based on the nucleic acid probe in the nucleic acid analyzing step and improve the detection sensitivity of the target nucleic acid.

In the above-mentioned second embodiment, in the nucleic acid analyzing step, the nucleic acids contained in the specimens and the identifier probes may be analyzed by using quantitative nucleic acid amplification assay or a base sequence reader capable of quantitative determination of the nucleic acids.

This makes it possible to perform more correct quantitative determination of the nucleic acids contained in the samples derived from the specimens respectively.

In the above-mentioned second embodiment, the nucleic acid contained in the identifier probe may be a DNA. The base length of the DNA is preferably from 20 bases or more but not more than 500 bases. The number of molecules of the DNA contained in each of the identifier probes is preferably 100 or more but not more than 100,000.

In the second embodiment, in the nucleic acid analyzing step, the DNA added to each of the specimens may be analyzed using quantitative nucleic acid amplification assay and the amplification rates of the DNAs in the quantitative nucleic acid amplification assay may be substantially equal to one another. The quantitative nucleic acid amplification assay is more preferably PCR (polymerase chain reaction) assay and a difference in amplification rate among the DNAS is preferably 1.9-fold or less, more preferably 1.1-fold or less.

This makes it possible to perform more correct quantitative determination of the DNA contained in the samples respectively derived from the specimens.

### [Advantageous Effects of Invention]

The present invention is effective for identifying the origin of each of a number of samples provided for analysis by making use of smaller kinds of nucleic acids.

### [Brief Description of the Drawings]

[FIG. 1] FIG. 1 is an entire constitution diagram of a nucleic acid analysis kit according to a first embodiment of the present invention.
[FIG. 2] FIG. 2 is a flow chart for describing a nucleic acid analysis method using the nucleic acid analysis kit of FIG. 1.
[FIG. 3] FIG. 3 is an entire constitution diagram of a nucleic acid analysis kit according to a second embodiment of the present invention.
[FIG. 4A] FIG. 4A is a view for describing a method of using a substrate which the nucleic acid analysis kit of FIG. 3 has while showing the substrate before division.
[FIG. 4B] FIG. 4B is a view for describing a method of using a substrate which the nucleic acid analysis kit of FIG. 3 has while showing the substrate after division.
[FIG. 5] FIG. 5 is a flow chart for describing a nucleic acid analysis method using the nucleic acid analysis kit of FIG. 3.
[FIG. 6] FIG. 6 is a flow chat for describing a nucleic acid analysis method according to a third embodiment of the present invention wherein the method is another nucleic acid analysis method using the nucleic acid analysis kit of FIG. 3.

### [Description of Embodiments]

### (First Embodiment)

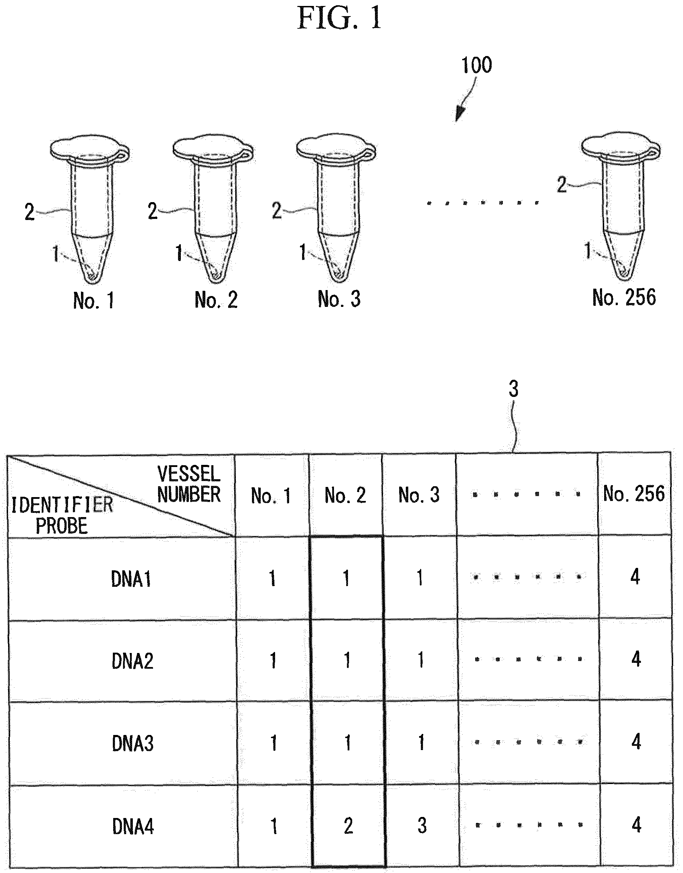

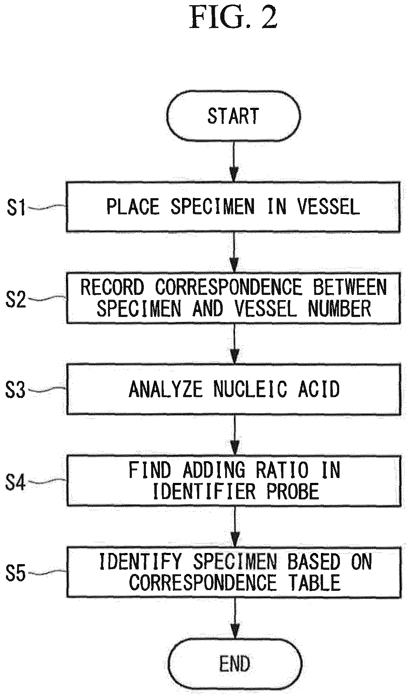

A nucleic acid analysis kit 100 according to a first embodiment of the present invention and a nucleic acid analysis method using this kit will hereinafter be described referring to FIGS. 1 and 2.

The nucleic acid analysis kit 100 according to the present embodiment has, as shown in FIG. 1, a plurality of vessels (supports) 2 having an identifier probe 1 sealed therein and a correspondence table 3 in which a vessel number given to each of the vessels 2 is associated with the composition of the identifier probe 1 sealed in each of the vessels 2.

Each of the vessels 2 is, for example, a 1.5-mL microtube ordinarily used in a molecular biological test. The identifier probe 1 is attached to the inner bottom surface of the vessel 2 in dry form.

The identifier probe 1 contains a plurality of kinds of nucleic acids having known base sequences different from each other. The plurality of kinds of nucleic acids is sealed in each of the vessels as a mixture obtained by adding them at ratios different for each of the vessels 2. The composition of the identifier probe 1, that is, the kind and amount of the nucleic acids contained in the identifier probe 1 is different for each of the vessels 2.

In the present embodiment, an example using four kinds of nucleic acids, that is, DNA1, DNA2, DNA3, and DNA4 will be described. DNA1, DNA2, DNA3, and DNA4 are added in four levels different from one another, respectively. This means that in the present example, the number of identifier probes 1 different from one another in adding ratio which are available in the present example is 4×4×4×4=256. The maximum number of the vessels 2 that the nucleic acid analysis kit 100 is able to have is 256.

The kind of nucleic acids contained in the identifier probe 1 can be selected as needed in accordance with the kind of a target nucleic acid of the specimen and another kind of nucleic acids such as RNA may be used. When the target nucleic acid is a DNA, the identifier probe 1 contains preferably a DNA. When the target nucleic acid is an RNA, on the other hand, the identifier probe 1 contains preferably an RNA. When the identifier probe 1 contains an RNA, a modified RNA, for example, an RNA obtained by substituting the 2'-group of an oligonucleotide with a methyl group may be used in order to prevent degradation of the RNA by a degrading enzyme.

DNA1 to DNA4 each have a base length of preferably 10 bases or more but not more than 1000 bases from the standpoint of handling ease in synthesis and analysis, with 20 bases or more but not more than 500 bases being more preferred. The total number of molecules of DNA1 to DNA4 to be sealed in each of the vessels 2 is preferably 100 or more but not more than 100,000 in order to secure sufficient quantitativeness of DNA1 to DNA4 while preventing DNA1 to DNA4 from having an influence on the analysis of a target nucleic acid in a nucleic acid analyzing step which will be described later. It is however needless to say that the total number of molecules of the identifier probe 1 can be set to fall outside the above range in accordance with the precision or sensitivity of a method of nucleic acid analysis.

The correspondence table 3 shows one-to-one correspondence between the composition of the identifier probe 1 in each of the vessels 2, that is, the kind of nucleic acids (DNA1 to DNA4) and an adding ratio of them as the kind and amount of nucleic acids contained in each identifier probe 1, and the vessel number of each of the vessels 2.

Next, a nucleic acid analysis method using the nucleic acid analysis kit 100 having such a constitution will be described referring to the flow chart of FIG. 2.

A target DNA contained in a specimen is analyzed by using the nucleic acid analysis kit 100 according to the present embodiment in the following manner. First, a specimen is placed in the vessel 2 and the specimen and the identifier probe 1 are mixed sufficiently in the vessel 2 (Step S1, probe adding step). At this time, which specimen is placed in the vessel 2 and the number of the vessel number of the vessels 2 in which the specimen is placed are recorded (Step S2).

Next, each of the specimens is subjected to pretreatment for nucleic acid analysis. For example, when the specimen is a fragment or cell of a biological tissue, treatment for extracting a DNA from the specimen is performed. Next, a sample obtained by the pretreatment of each of the specimens is analyzed using quantitative nucleic acid amplification assay such as quantitative PCR to detect and quantitatively determine a target DNA contained in the sample (Step S3, nucleic acid analyzing step).

Each of the samples thus obtained has both the identifier probe 1 sealed in the vessel 2 and the target DNA derived from the specimen. In Step S3, detection and quantitative determination performed for the target DNA are carried out also for four kinds of DNAS, that is, DNA1 to DNA4 constituting the identifier probe 1. The four kinds of DNAs, that is, DNA1 to DNA4 therefore each have a base sequence as amplified by a primer for the target DNA or a primer for DNA1 to DNA4 is added in Step S3. As a result, an adding ratio of DNA1 to DNA4 of each of the samples can be found (Step S4).

In the quantitative nucleic acid amplification assay in Step S3, DNA1 to DNA4 in each of the samples is preferably amplified at a substantially equal amplification rate. More specifically, a difference in the amplification rate among DNA1 to DNA4 is preferably 1.9-fold or less, more preferably 1.1-fold or less.

Next, the adding ratio of DNA1 to DNA4 in each of the samples found in Step S4 is checked against the correspondence table 3. As a result, it is possible to identify the vessel number of the vessels 2 containing the specimen from which each of the samples is derived (Step S5).

Thus, according to the present embodiment, analysis of the identifier probe 1 contained in the sample prepared from each of the specimens enables identification of the origin of the sample. In molecular biological tests, during a period between placement of a specimen in the vessel 2 and completion of the analysis of a target DNA, the specimen or a sample obtained from the specimen is ordinarily transferred from one vessel to another vessel in a plurality of times. The present embodiment is advantageous in that without recording, at the time of transferring the samples, which sample is transferred and to which vessel the sample is transferred, the origin of the sample can be identified correctly based on the adding ratio in the identifier probe 1 contained in the sample provided for nucleic acid analysis finally.

The present embodiment is further advantageous in that by differentiating the adding amount of a plurality of kinds of nucleic acids, many kinds of identifier probes 1 which can be distinguished from one another can be prepared easily even though the kinds of nucleic acids used are sufficiently small and as a result, the origin of a number of samples can be identified.

In the present embodiment, quantitative nucleic acid amplification assay is used for nucleic acid analysis, but a method of nucleic acid analysis is not limited insofar as it permits detection and quantitative determination of a specific nucleic acid contained in a sample. A base sequence reader (DNA sequencer) having a quantitative determination function of nucleic acids may therefore be used instead of the quantitative nucleic acid amplification assay.

Even by such a method, an adding ratio of DNA1 to DNA4 constituting the identifier probe 1 can be found simultaneously with detection and quantitative determination of a target DNA contained in a sample.

### (Second Embodiment)

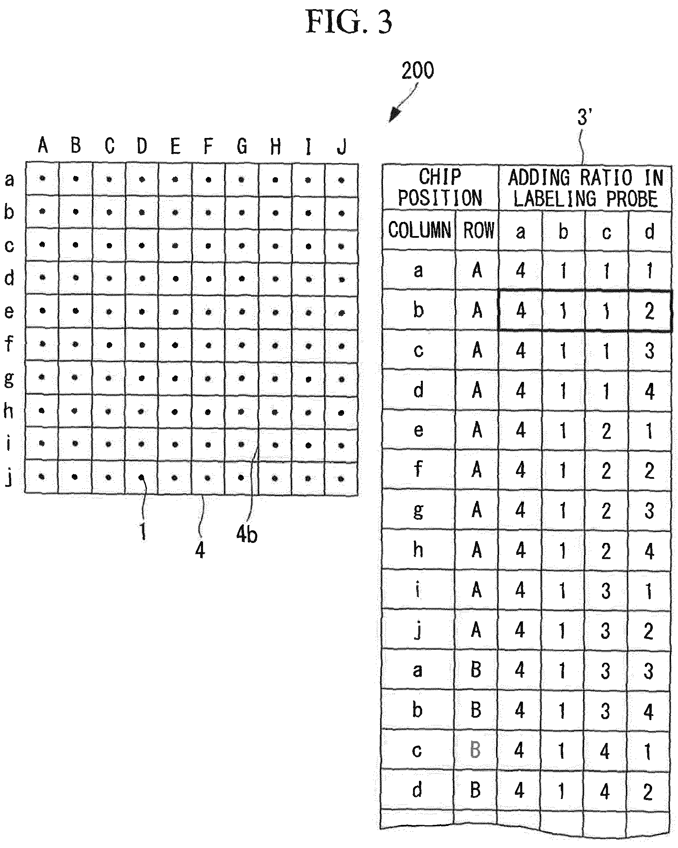

Next, a nucleic acid analysis kit 200 according to a second embodiment of the present invention and a nucleic acid analysis method using the kit will be described referring to FIGS. 3 to 5.

In the present embodiment, a difference from the first embodiment will mainly be described and constituents similar to those in the first embodiment will be identified by the same symbols and a description on them is omitted.

The nucleic acid analysis kit 200 according to the present embodiment is different from the kit according to the first embodiment in that it has a substrate 4 having a plurality of kinds of identifier probes 1 attached thereto instead of the vessels 2 as shown in FIG. 3 and in a correspondence table 3', the composition of each of the identifier probes 1 and an attaching position of them on the substrate 4 are associated with each other.

The substrate 4 is a flat plate, such as cover glass, suited for attaching thereto a section cut out from a biological tissue. The substrate 4 has a division line 4a for dividing the surface of the substrate into a plurality of regions in grid form. The regions obtained by dividing the substrate along the division line 4a each have a single kind of the identifier probe 1 attached and the adding ratio of DNA1 to DNA4 contained in the identifier probe 1 differs for each of the regions. The identifier probes 1 are attached to the substrate 4, for example, by using an inkjet printer or a spotter to be used in the manufacture of DNA chips.

In the present embodiment, similar to the first embodiment, it is assumed that four kinds of DNAS, that is, DNA1, DNA2, DNA3, and DNA4 are mixed by adding them in four levels different from one another. Accordingly, the substrate 4 may be divided into regions not more than 256 by the division line 4a. After division, the substrate 4 shown in FIG. 3 has 100 regions, that is, 10 rows × 10 columns.

The correspondence table 3' shows one-to-one correspondence between the position of each of the regions and an adding ratio of each of DNA1 to DNA4 which the identifier probes 1 attached to each of the regions has. The position of each of the regions can be identified by row numbers a, b, c, ..., j and column numbers A, B, ..., J.

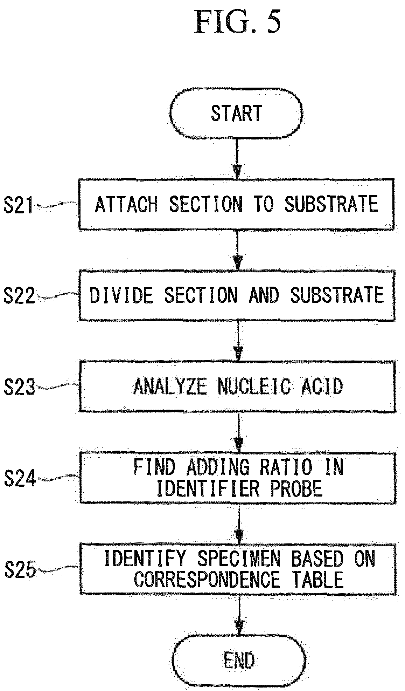

Next, a nucleic acid analysis method using the nucleic acid analysis kit 200 having such a constitution will be described referring to the flow chart of FIG. 5.

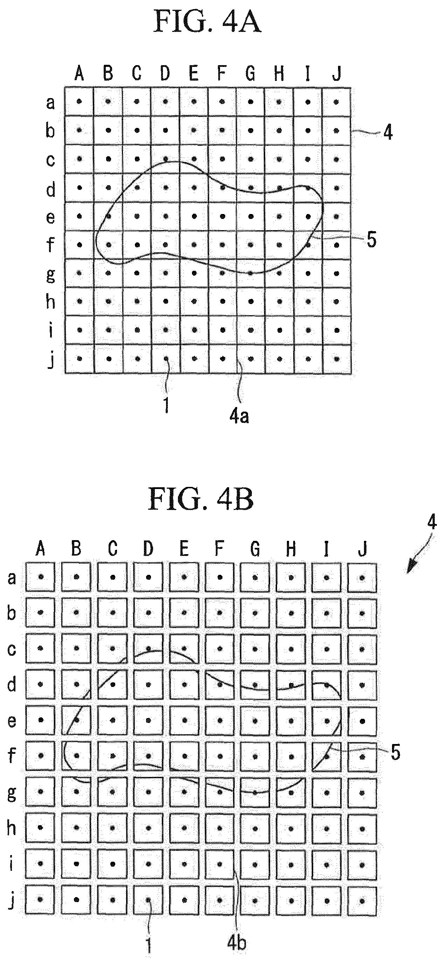

A nucleic acid contained in a section 5 of a biological tissue is analyzed by using the nucleic acid analysis kit 200 according to the present embodiment in the following manner. First, as shown in FIG. 4A, the section 5 cut out from a biological tissue is attached to the substrate 4 (Step S21, probe adding step). Next, as shown in FIG. 4B, the substrate 4 and the section 5 are cut along the division line 4a to obtain 100 chips (supports) 4b having a fragment of the section 5, which will be a specimen, attached to at least some of the chips (Step S22, dividing step).

A method of cutting the substrate 4 and the section 5 is not particularly limited. For example, after pre-cut chips 4b are arranged two-dimensionally on a stretch sheet to prepare the substrate 4 and the section 5 is attached onto the substrate 4, the resulting sheet is stretched to separate the chips 4b from one another. In such a manner, the substrate 4 and the section 5 can be cut simultaneously along the division line 4a made of a boundary between the chips 4b.

The chips 4b thus obtained are, for example, placed in microtubes, respectively, and the fragment of the section 5 attached to each of the chips 4b is subjected to pretreatment such as nucleic acid extraction. The sample obtained from each of the specimens by the pretreatment is subjected to nucleic acid analysis similar to Step S3 of the first embodiment to detect and quantitatively determine a target DNA contained in the sample (Step S23, nucleic acid analyzing step).

Each of the samples prepared from the fragment of the section 5 has both the single kind of the identifier probe 1 attached to the substrate 4 and a target DNA derived from the fragment. Similar to the above-mentioned steps S3 to S5, an adding ratio of DNA1 to DNA4 of each of the samples can be found (Step S24) and the resulting adding ratio of DNA1 to DNA4 in each of the samples is checked against the correspondence table 3', whereby the position of the fragment from which each of the samples is derived can be identified (Step S25).

The present embodiment has an advantage similar to that of the first embodiment so that a description of it is omitted.

It is to be noted that in the present embodiment, the identifier probe 1 is attached to each region of the substrate 4 but alternatively, after the section 5 is attached to the substrate 4, the identifier probe 1 may be attached onto the section 5 by using the above-mentioned inkjet printer, spotter, or the like. Also in this case, the attaching position of the identifier probe 1 is determined so that regions divided by the division line 4a each include a single kind of the identifier probe 1.

In such a manner, it is also possible to correctly identify the position of the fragment of the section 5 from which each of the samples provided for nucleic acid analysis is derived, based on the composition of the identifier probe 1 contained in each of the samples.

### (Third Embodiment)

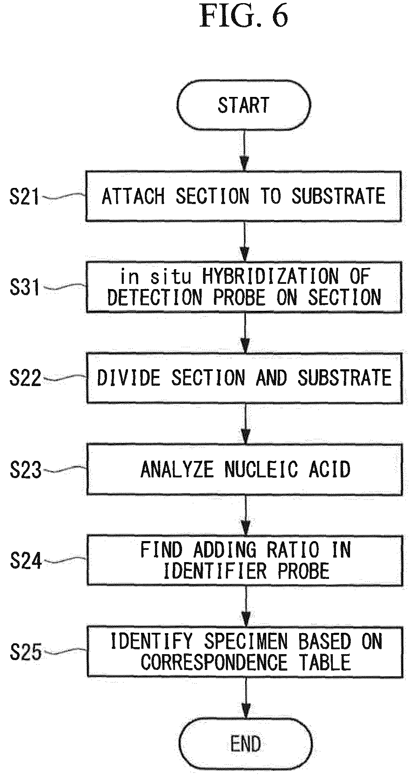

Next, a nucleic acid analysis kit according to a third embodiment of the present invention and a nucleic acid analysis method using the kit will be described referring to FIG. 6. The present embodiment relates to another nucleic acid analysis method using the nucleic acid analysis kit 200 according to the second embodiment. A difference from the second embodiment will therefore be described mainly and constituents similar to those in the second embodiment will be identified by the same symbols and a description of them is omitted.

In the nucleic acid analysis method according to the present embodiment, as shown in FIG. 6, after the section 5 is attached to the substrate 4 in Step S21, in situ hybridization is performed to associate a DNA probe for detection (nucleic acid probe which will hereinafter be called "detection probe") with a target RNA on the section 5 (Step S31, hybridization step). After the detection probe is associated, the section 5 is divided and collected (Step S22), nucleic acid analysis is conducted (Step S23), an adding ratio in the identifier probe 1 is found (Step S24), and the specimen is identified based on the correspondence table 3' (Step S25) similar to the second embodiment. In the present embodiment, however, in Step S23, the detection probe is also analyzed and in Step S24, the kind and amount of the detection probe contained in each of the specimens are also found.

When instead of using the substrate 4 to which the identifier probe 1 has been attached in advance, the identifier probe 1 is attached using an inkjet printer or the like after attachment of the section 5 to the substrate 4, hybridization in Step S31 is followed by attachment of the identifier probe 1 to the section 5.

According to the nucleic acid analysis method of the present embodiment, improved detection accuracy can be achieved in addition to the advantage of the second embodiment, because when a target nucleic acid is analyzed using quantitative nucleic acid amplification assay, a known detection probe is used to make the amplification rate substantially equal to each other. In particular, when the target nucleic acid is an RNA, analysis can be conducted using quantitative nucleic acid amplification assay and a DNA sequencer without replacing the RNA with a complementary DNA upon nucleic acid analysis. Further, improvement in detection accuracy and sensitivity can be achieved by detecting while replacing a readily degradable RNA to a DNA.

In the above-mentioned first to third embodiments, the identifier probe 1 containing four kinds of nucleic acids has been described. The number of the kinds of the nucleic acids contained in the identifier probe 1 is not limited thereto. It is preferably the number suited for practical use. For example, in the second embodiment, the number is preferably suited for use of with an inkjet printer or spotter. More specifically, the number of the kinds of nucleic acids contained in the identifier probe 1 is, when a four-color inkjet printer is used, preferably 4 or less. When a spotter is used, the number is preferably not more than the number of pins which the spotter has.

In the above-mentioned first to third examples, in order to find a correct adding ratio of DNA1, DNA2, DNA3, and DNA4 by nucleic acid analysis, the respective adding amounts of DNA1, DNA2, DNA3, and DNA4 are made different, more specifically, are made 2-fold, 3-fold, and 4-fold supposing that the minimum adding amount is 1. When the amount of DNA1, DNA2, DNA3, and DNA4 contained in the sample can be detected with high accuracy, however, DNA1, DNA2, DNA3, and DNA4 may be added at a reduced change ratio.

In the above-mentioned first to third embodiments, the identifier probe 1 contains a plurality of nucleic acids. In accordance with the conditions such as the number of specimens to be analyzed or determination accuracy of nucleic acid analysis, the identifier probe 1 may contain only a single kind of a nucleic acid.

For example, in the case of the first embodiment, when the nucleic acid analysis kit 100 has n pieces (n is a natural number) of vessels 2, an identifier probe 1 containing only a single kind of a nucleic acid is placed in each of the vessels 2 and the amount of this nucleic acid may be varied in n stages for each of the vessels 2.

Even under such conditions, by analyzing the identifier probe 1 in the sample provided for nucleic acid analysis, the origin of the sample can be identified.

### [Reference Signs List]

- 100, 200:: nucleic acid analysis kit
- 1:: identifier probe
- 2:: vessel (support)
- 3, 3':: correspondence table
- 4:: substrate
- 4a:: division line
- 4b:: chip (support)
- 5:: section
- S1, S21:: probe adding step
- S3, S23:: nucleic acid analyzing step
- S22:: dividing step
- S31:: hybridization step

## Claims

1. A nucleic acid analysis kit, comprising:
a plurality of supports which retain identifier probes containing a nucleic acid and support specimens, respectively,
wherein the identifier probes each contain at least one kind of a nucleic acid having a known base sequence, are different from one another in at least one of the kind and amount of the nucleic acid for each of the supports, and are retained by the supports so as to be miscible with the specimens respectively.

2. The nucleic acid analysis kit according to Claim 1,
further comprising a correspondence table in which each of the plurality of supports is associated with a combination of the kind and amount of the nucleic acid contained in the identifier probe retained by each of the plurality of supports.

3. The nucleic acid analysis kit according to Claim 1 or Claim 2,
wherein the supports are vessels for having therein the specimens, and
the identifier probes have been sealed in the vessels.

4. The nucleic acid analysis kit according to Claim 1 or Claim 2, further comprising:
a substrate which can be divided into a plurality of chips along predetermined division lines and to which a section of a biological tissue as the specimen is to be attached, and
wherein the identifier probe is retained by each of the plurality of chips.

5. The nucleic acid analysis kit according to any of Claim 1 to Claim 4, wherein the nucleic acid is a DNA.

6. The nucleic acid analysis kit according to Claim 5,
wherein the DNA has a base length of 20 bases or more but not more than 500 bases.

7. The nucleic acid analysis kit according to Claim 5 or Claim 6,
wherein the number of molecules of the DNA retained by each of the supports is 100 or more but not more than 100,000.

8. A nucleic acid analysis method, comprising:
a probe adding step in which identifier probes each containing at least one nucleic acid having a known base sequence are added to a plurality of specimens, respectively, and
a nucleic acid analyzing step in which the nucleic acid contained in each of the specimens is analyzed,
wherein in the probe adding step, the identifier probes different from each other in at least one of the kind and amount of the nucleic acid are added to the specimens, and
in the nucleic acid analyzing step, the nucleic acid contained in each of the identifier probes respectively added to the specimens is also analyzed.

9. The nucleic acid analysis method according to Claim 8,
wherein in the probe adding step, the specimens are added to vessels having the identifier probes therein, respectively.

10. The nucleic acid analysis method according to Claim 8,
wherein in the probe adding step, a section of a biological tissue is attached onto a substrate having the identifier probes attached thereto in scattered form,
wherein further comprises a dividing step in which the substrate having the section attached thereto is divided with the section into a plurality of chips each containing one of the identifier probes, and
wherein in the nucleic acid analyzing step, a portion of the section attached to each of the chips obtained in the dividing step is analyzed.

11. The nucleic acid analysis method according to Claim 8,
wherein in the probe adding step, the identifier probes are attached in scattered form onto a section of a biological tissue,
wherein further comprises a dividing step in which the section having the identifier probes attached thereto is divided into a plurality of fragments containing one of the identifier probes, and
wherein in the nucleic acid analyzing step, the fragments of the section obtained by cutting in the dividing step are analyzed.

12. The nucleic acid analysis method according to Claim 10 or Claim 11, further comprising prior to the dividing step:
a hybridization step in which the section is subjected to in situ hybridization using a nucleic acid probe having a known base sequence complementary to a target nucleic acid.

13. The nucleic acid analysis method according to any of Claim 8 to Claim 12,
wherein in the nucleic acid analyzing step, the nucleic acids contained in the specimens and the identifier probes are analyzed by quantitative nucleic acid amplification assay or a base sequence reader capable of quantitative determination of nucleic acids.

14. The nucleic acid analysis method according to any of Claim 8 to Claim 13,
wherein the nucleic acid contained in the identifier probes is a DNA.

15. The nucleic acid analysis method according to Claim 14,
wherein the DNA has a base length of 20 bases or more but not more than 500 bases.

16. The nucleic acid analysis method according to Claim 14 or Claim 15,
wherein the number of molecules of the DNA contained in each of the identifier probes is 100 or more but not more than 100,000.

17. The nucleic acid analysis method according to any of Claim 14 to Claim 16,
wherein in the nucleic acid analyzing step, the DNA added to each of the specimens is analyzed using quantitative nucleic acid amplification assay, and
amplification rates of the DNAs in the quantitative nucleic acid amplification assay are substantially equal to one another.

18. The nucleic acid analysis method according to Claim 17,
wherein in the nucleic acid analyzing step, the DNA added to each of the specimen is analyzed using PCR, and
a difference in amplification rate among the DNAs in the PCR is 1.9-fold or less.
